# EUROPEAN PATENT SPECIFICATION

(11) **EP 4 557 434 B1**
(45) Date of publication and mention of the grant of the patent: **07.10.2026**
(21) Application number: 23907568.2
(22) Date of filing: 13.12.2023
(51) Int. Cl.: H01M 10/0567, H01M 10/0569, H01M 10/052, H01M 4/38, H01M 10/0568, H01M 10/0564, C07D 213/71, C07D 341/00, H01M 4/58, H01M 4/62, H01M 10/42, H01M 4/36, H01M 4/40

(54) **ELECTROLYTE SOLUTION FOR LITHIUM SECONDARY BATTERY AND LITHIUM SECONDARY BATTERY COMPRISING THE SAME**
ELEKTROLYT FÜR LITHIUMSEKUNDÄRBATTERIE UND LITHIUMSEKUNDÄRBATTERIE DAMIT
ÉLECTROLYTE POUR BATTERIE SECONDAIRE AU LITHIUM ET BATTERIE SECONDAIRE AU LITHIUM LE COMPRENANT

(30) Priority: 23.12.2022 KR 20220183773
(43) Date of publication of application: 21.05.2025
(73) Proprietor: LG Energy Solution, Ltd., Seoul 07335 (KR)
(72) Inventor: AHN, Ji-Hoon, Daejeon 34122 (KR); JANG, Eun-Ji, Daejeon 34122 (KR)
(74) Representative: Goddar, Heinz J.
(86) International application number: PCT/KR2023/020567
(87) International publication number: WO 2024/136282

(56) References cited:
- CN-A- 109 244 542
- CN-B- 110 148 787
- JP-A- 2001 307 766
- KR-A- 20140 001 693
- KR-A- 20210 050 133
- KR-A- 20210 050 133
- KR-A- 20210 113 055
- KR-A- 20210 113 055

## Description

### TECHNICAL FIELD

The present disclosure relates to an electrolyte solution for a lithium secondary battery and a lithium secondary battery comprising the same.

More particularly, the present disclosure relates to an electrolyte solution suitable for use in a lithium-sulfur battery and a lithium-sulfur battery comprising the same.

The present application claims priority to Korean Patent Application No. 10-2022-0183773 filed on December 23, 2022 in the Republic of Korea.

### BACKGROUND ART

A lithium-sulfur battery is a battery system using a sulfur-based material having a sulfur-sulfur (S-S) bond as a positive electrode active material and a lithium metal as a negative electrode active material. Sulfur, a main component of the positive electrode active material, is abundant in nature and can be found around the world, is non-toxic, and has low atomic weight.

As secondary batteries are used in a wide range of applications including electric vehicles (EVs) and energy storage systems (ESSs), attention is drawn to lithium-sulfur battery technology that achieves higher energy storage density by weight (~2,600 Wh/kg) than lithium-ion secondary batteries having lower energy storage density by weight (~250 Wh/kg).

During discharging, lithium-sulfur batteries undergo oxidation at the negative electrode active material, lithium, by releasing electrons into lithium cation, and reduction at the positive electrode active material, the sulfur-based material, by accepting electrons. Through the reduction reaction, the sulfur-based material is converted to sulfur anion by the S-S bond accepting two electrons. The lithium cation produced by the oxidation reaction of lithium migrates to the positive electrode via an electrolyte, and bonds with the sulfur anion produced by the reduction reaction of the sulfur-based compound to form a salt. Specifically, sulfur before the discharge has a cyclic S₈ structure, and it is converted to lithium polysulfide (LiSx) by the reduction reaction and is completely reduced to lithium sulfide (Li₂S).

In this instance, it is difficult to ensure reactivity with electrons and lithium ions in solid state due to low electrical conductivity of sulfur in the sulfur-based compound as the positive electrode active material. Accordingly, to improve reactivity of sulfur in lithium-sulfur batteries, technology has been developed to produce intermediate polysulfide Li₂Sₓ, to induce liquid phase reaction and improve reactivity. This technology uses, as solvents of electrolyte solutions, ether-based solvents in which lithium polysulfide highly dissolves, for example, dioxolane, dimethoxy ethane (DME), etc. Thus, the amount of electrolyte solution affects reactivity of sulfur and battery life.

Additionally, polysulfide (PS) produced from the positive electrode during discharging of lithium-sulfur batteries is eluted into electrolyte solutions, causing reaction, and the polysulfide causes side reaction in the batteries, resulting in battery degradation. Despite high theoretical density, energy density reduces rapidly over the repeated charge and discharge cycles. KR 2021 0113055 A discloses a lithium-sulfur battery electrolyte comprising a non-aqueous organic solvent system containing an ether-based compound and a double-bond-containing heterocyclic compound, optionally together with nitrate/nitrite-based compounds and sulfide additives, to suppress polysulfide-related side reactions, stabilize the lithium metal electrode, and improve the cycling performance and lifespan of lithium-sulfur batteries. KR 2021 0050133 A discloses a lithium-sulfur battery electrolyte comprising a lithium salt and a non-aqueous solvent system containing disulfide-based solvent, wherein the disulfide-based solvent may include aryl/heteroaryl disulfide compounds to reduce electrolyte viscosity, enhance ionic conductivity, improve sulfur utilization, and increase the discharge capacity and energy performance of lithium-sulfur batteries.

Accordingly, there is a need for technology for suppressing capacity fading over repeated charge and discharge cycles and improving capacity retention.

### SUMMARY

### Technical Problem

The inventors aim to provide a lithium-sulfur battery with suppressed capacity fading over repeated charge and discharge cycles and improved capacity retention.

To this end, the present disclosure is directed to providing an electrolyte solution for a lithium-sulfur battery , according to Claim 1, for changing reaction pathways of sulfur (S) to control the elution of polysulfide (PS) in order to solve the issue raising from the elution of polysulfide, for example, battery capacity fading.

Therefore, the present disclosure is directed to providing a lithium-sulfur battery, according to Claim 11, with improved capacity retention and high energy efficiency.

### Advantageous Effects

The electrolyte solution for the lithium-sulfur battery according to an embodiment of the present disclosure may change reduction reaction pathways of lithium sulfide to change the discharge mechanism of the lithium-sulfur battery.

In particular, it may be possible to suppress capacity fading of the battery caused by the elution of lithium polysulfide into the electrolyte solution, thereby slowing down the degradation rate of the battery over repeated charge and discharge cycles, and accordingly improving Coulombic efficiency of the battery and capacity retention performance of the battery.

Accordingly, according to an embodiment of the present disclosure, it may be possible to provide the lithium-sulfur battery having high energy density, for example, energy density of 300 Wh/kg or more, and further, 400 Wh/kg or more.

### BRIEF DESCRIPTION OF THE DRAWINGS

FIG. 1 shows measurement results of the number of cycles at the time when 90% capacity retention is reached over repeated charge and discharge cycles in Example 1, Example 2 and Comparative Example 1.
FIG. 2 shows changes in Coulombic efficiency per weight as a function of charge and discharge cycle in Example 1, Example 2 and Comparative Example 1.
FIG. 3 shows measurement results of the number of cycles at the time when 90% capacity retention is reached over repeated charge and discharge cycles in Example 1, Comparative Example 3 and Comparative Example 4.

### DETAILED DESCRIPTION

Hereinafter, the present disclosure will be described in more detail.

It should be understood that the terms or words used in the specification and the appended claims should not be construed as limited to general and dictionary meanings, but rather interpreted based on the meanings and concepts corresponding to the technical aspect of the present disclosure on the basis of the principle that the inventor is allowed to define terms appropriately for the best explanation.

The terminology used herein is for the purpose of describing particular embodiments only and is not intended to be limiting of the present disclosure. As used herein, the singular forms include the plural forms as well, unless the context clearly indicates otherwise. It should be understood that the term "comprise" or "include" when used in this specification, specifies the presence of stated features, integers, steps, operations, elements, components or a combination thereof, but does not preclude the presence or addition of one or more other features, integers, steps, operations, elements, components or a combination thereof.

The term "composite" as used herein refers to a material with physically·chemically different phases and more effective functions, formed by combining two or more materials.

The term "(poly)sulfide" as used herein is the concept that covers "(poly)sulfide ion (Sₓ²⁻, 1≤x≤8)" and "lithium (poly)sulfide (Li₂Sₓ or Li₂Sₓ⁻ 1≤x≤8)".

The term "polysulfide" as used herein is the concept that covers "polysulfide ion (Sₓ²⁻, 1<x≤8)" and "lithium polysulfide (Li₂Sₓ or Li₂Sₓ⁻ 1<x≤8)".

A lithium secondary battery, especially a lithium-sulfur battery suffers from rapid capacity fading over repeated charge and discharge cycles due to the shuttle effect of lithium polysulfide eluted from a positive electrode into an electrolyte during charging and discharging.

An aspect of the present disclosure is intended to solve the above-described problem.

### Electrolyte solution for lithium secondary battery

Accordingly, the present disclosure relates to an electrolyte solution for a lithium secondary battery for solving the above-described problem.

The electrolyte solution for the lithium secondary battery of the present disclosure includes a nonaqueous solvent, a lithium salt, nitrate and an aryl derivative.

The nonaqueous solvent includes a linear ether compound and a conjugated heterocyclic compound.

The aryl derivative includes a compound represented by the following Chemical Formula 1, a compound represented by the following Chemical Formula 2, a compound represented by the following Chemical Formula 3 or a mixture thereof.

[Chemical Formula 1] R₁-S⁻

[Chemical Formula 2] R₂-S-H

[Chemical Formula 3] R₃-S-S-R₄

In the above Chemical Formulas 1 to 3, each of R₁ to R₄ is independently a 5- or 6-membered heteroaryl containing at least one N or S atom as a ring atom.

In the present disclosure, the compound represented by the above Chemical Formula 1 may typically include a thiolate compound.

In the present disclosure, the compound represented by the above Chemical Formula 2 may typically include a thiol compound.

In the present disclosure, the compound represented by the above Chemical Formula 3 may typically include a disulfide compound.

According to an aspect of the present disclosure, the aryl derivative may suppress irreversible reaction of lithium polysulfide eluted from a positive electrode of the lithium-sulfur battery into an electrolyte solution, thereby effectively suppressing capacity fading of the battery caused by the shuttle effect of lithium polysulfide.

In the present disclosure, the heteroaryl is an aromatic cyclic compound and refers collectively to organic compounds including atoms of other elements than carbon as ring atoms. In particular, in the present disclosure, each of R₁ to R₄ is independently a 5- or 6-membered heteroaryl containing at least one N or S atom as a ring atom.

In an embodiment of the present disclosure, each of the heteroaryls R₁ to R₄ may be bonded to the -S⁻, -SH or -S-S- functional group, and in this instance, each of the functional groups may be independently substituted in place in the heteroaryl at the ortho-, meta- or para-position of the hetero ring atom.

In another embodiment of the present disclosure, each of the heteroaryls R1 to R4 may be bonded to the -S⁻, -SH or -S-S- functional group, and in this instance, each of the functional groups may be independently substituted in place in the heteroaryl at the ortho-position of the hetero ring atom.

According to an embodiment of the present disclosure, each of R₁ to R₄ may be independently an ortho-pyridyl group.

In an embodiment of the present disclosure, the aryl derivative may include 2,2'-dipyridyl disulfide.

In an embodiment of the present disclosure, the electrolyte solution for the lithium-sulfur battery may include the aryl derivative in amount of 0.1 wt% to 5 wt% based on the total weight of the electrolyte solution for the lithium-sulfur battery. For example, the aryl derivative may be included in an amount of 0.1 wt% to 3 wt%, or 0.5 wt% to 3 wt%, 0.5 wt% to 2 wt%, 0.5 wt% to 1.5 wt% or 1.0 wt% to 1.5 wt% based on the total weight of the electrolyte solution for the lithium-sulfur battery. When the amount of the aryl derivative is within the above-described range, the aryl derivative may be beneficial in terms of efficiency of capacity retention improvement of the lithium-sulfur battery, but the present disclosure is not limited thereto.

In an embodiment of the present disclosure, a weight ratio of the aryl derivative to the nitrate (the aryl derivative/the nitrate) may be 0.2 to 1.5. Specifically, the weight ratio of the aryl derivative to the nitrate (the aryl derivative/the nitrate) may be 0.2 to 1.0, 0.2 to 0.5, 0.2 to 0.3, 0.25 to 0.5 or 0.25 to 0.3. When the weight ratio of the aryl derivative to the nitrate is within the above-described range, the aryl derivative may be beneficial in terms of efficiency of capacity retention improvement of the lithium-sulfur battery, but the present disclosure is not limited thereto.

In addition to the lithium salt, the nitrate may provide ions when it dissolves in the electrolyte solution of the lithium secondary battery, thereby improving electrical conductivity of the lithium secondary battery and improving life characteristics of the battery when the electrolyte solution for the lithium secondary battery is used in the lithium-sulfur battery. Specifically, the efficacy of the nitrate is not limited thereto, but for example, the nitrate may suppress reduction reaction of lithium polysulfide during charging and discharging of the lithium-sulfur battery, thereby preventing irreversible consumption of lithium polysulfide, leading to improved performance of the lithium-sulfur battery.

In an embodiment of the present disclosure, the nitrate may include, without limitation, any type of nitrate that forms a stable coating on the negative electrode, i.e., the lithium metal electrode of the lithium secondary battery, specifically the lithium-sulfur battery and improves charge and discharge efficiency, and may include, for example, nitric acid-based compounds, nitrous acid-based compounds or a mixture thereof.

In an embodiment of the present disclosure, the nitrate may be, for example, selected from the group consisting of inorganic nitric acid-based or nitrous acid-based compounds such as lithium nitrate (LiNO₃), potassium nitrate (KNO₃), cesium nitrate (CsNO₃), barium nitrate (Ba(NO₃)₂), ammonium nitrate (NH₄NO₃), lithium nitrite (LiNO₂), potassium nitrite (KNO₂), cesium nitrite (CsNO₂), ammonium nitrite (NH₄NO₂); organic nitric acid-based or nitrous acid-based compounds such as methyl nitrate, dialkyl imidazolium nitrate, guanidine nitrate, imidazolium nitrate, pyridinium nitrate, ethyl nitrite, propyl nitrite, butyl nitrite, pentyl nitrite, octyl nitrite; organic nitro compounds such as nitromethane, nitropropane, nitrobutane, nitrobenzene, dinitrobenzene, nitropyridine, dinitropyridine, nitrotoluene, dinitrotoluene and a combination thereof, but is not limited thereto.

In an embodiment of the present disclosure, the cation of the nitrate may be selected from alkali metals, for example, lithium, sodium, potassium, rubidium and cesium, but is not limited thereto.

In another embodiment of the present disclosure, the nitrate may include lithium nitrate (LiNO₃).

In an embodiment of the present disclosure, the nitrate may be, for example, included in an amount of 1 wt% to 10 wt%, 2 wt% to 10 wt% or 3 wt% to 10 wt%, specifically 3 wt% to 8 wt%, 3 wt% to 6 wt% or 3 wt% to 5 wt% based on the total weight of the electrolyte solution for the lithium secondary battery, but is not limited thereto. When the nitrate is included in the above-described amount, the nitrate may have a beneficial effect on electrical conductivity of the electrolyte solution and polysulfide reduction suppression when used in the lithium-sulfur battery, but the present disclosure is not limited thereto.

According to an aspect of the present disclosure, the nonaqueous solvent is a medium for the movement of ions involved in the electrochemical reaction of the lithium secondary battery, and is used to dissolve the lithium salt, the nitrate and/or the aryl derivative.

To achieve the above-described effect, according to the present disclosure, the nonaqueous solvent includes a linear ether compound and a conjugated heterocyclic compound.

In an embodiment of the present disclosure, the nonaqueous solvent may include the linear ether compound and the conjugated heterocyclic compound in an amount of 80 vol% or more, for example, 85 vol% to 100 vol%, 90 vol% to 100 vol%, 95 vol% to 100 vol%, 98 vol% to 100 vol%, 90 vol% to 98 vol%, or 90 vol% to 95 vol% based on the total volume of the nonaqueous solvent. When the amount of the two types of compounds is within the above-described range based on the total volume of the nonaqueous solvent, it may have a beneficial effect on solubility of the lithium salt, the nitrate and the aryl derivative, but the present disclosure is not limited thereto.

**In** an embodiment of the present disclosure, in addition to the linear ether, the nonaqueous solvent may further include a cyclic ether.

**In** an embodiment of the present disclosure, the linear ether may include, for example, at least one selected from the group consisting of dimethyl ether, diethyl ether, dipropyl ether, dibutyl ether, diisobutyl ether, ethylmethyl ether, ethylpropyl ether, ethyltertbutyl ether, dimethoxymethane, trimethoxymethane, dimethoxyethane, diethoxyethane, dimethoxypropane, diethyleneglycol dimethylether, diethyleneglycol diethylether, triethyleneglycol dimethylether, tetraethyleneglycol dimethylether, ethyleneglycol divinylether, diethyleneglycol divinylether, triethyleneglycol divinylether, dipropylene glycol dimethyl ether, butylene glycol ether, diethyleneglycol ethylmethylether, diethyleneglycol isopropylmethylether, diethyleneglycol butylmethylether, diethyleneglycol tertbutylethylether and ethyleneglycol ethylmethylether. Preferably, the linear ether may include at least one selected from the group consisting of dimethylether, dimethoxyethane, diethoxyethane, diethyleneglycol dimethylether, triethyleneglycol dimethylether and tetraethyleneglycol dimethylether, and more preferably, dimethoxyethane.

In an embodiment of the present disclosure, the conjugated heterocyclic compound refers collectively to compounds having a structure in which p orbitals of 3 or more neighboring atoms of the compound and other p orbitals of adjacent atoms connected with a sigma bond may overlap, and other atom than carbon is included in the structure as a ring atom.

In an embodiment of the present disclosure, as the conjugated heterocyclic compound is included as the nonaqueous solvent, the heterocyclic compound forms a polymer passivation layer (a solid electrolyte interphase (SEI) layer) on the surface of lithium-based metal (a negative electrode) by ring opening reaction at the initial discharge stage of the battery, thereby suppressing lithium dendrite growth. Further, it may reduce electrolyte decomposition on the lithium-based metal surface and its consequential side reaction, leading to longer life of the lithium-sulfur battery. Additionally, it is difficult to dissolve the salt due to delocalization of lone pair electrons of the heteroatom, typically the sulfur atom by the conjugated structure, thereby reducing the amount of polysulfide eluted into the electrolyte solution.

In an embodiment of the present disclosure, the conjugated heterocyclic compound may be 4- to 15-, preferably 4- to 7-, and more preferably 5- to 6-membered heterocyclic compounds. Additionally, the conjugated heterocyclic compound may be a substituted or unsubstituted heterocyclic compound with at least one selected from the group consisting of an alkyl group having 1 to 4 carbon atoms, a cyclic alkyl group having 3 to 8 carbon atoms, an aryl group having 6 to 10 carbon atoms, a halogen group, a nitro group (-NO₂), an amine group (-NH₂) or a sulfonyl group (-SO₂). Additionally, the conjugated heterocyclic compound may be a polycyclic compound of the heterocyclic compound and at least one of a cyclic alkyl group having 3 to 8 carbon atoms or an aryl group having 6 to 10 carbon atoms.

When the conjugated heterocyclic compound is substituted with an alkyl group having 1 to 4 carbon atoms, radicals are stabilized, thereby suppressing side reaction derived from decompose of the electrolyte solution. Additionally, when the conjugated heterocyclic compound is substituted with a halogen group or a nitro group, a functional protective layer may be preferably formed on the lithium-based metal surface, and in this instance, the functional protective layer may be stable as a compact protective layer, and may allow uniform deposition of the lithium-based metal and suppress side reaction between polysulfide and the lithium-based metal.

In an embodiment of the present disclosure, the conjugated heterocyclic compound may include at least one of a conjugated cyclic ether compound or a thiophene-based compound.

In an embodiment of the present disclosure, the conjugated cyclic ether compound may include, for example, a furan-based compound and a pyran-based compound, and more specifically, furan, 2-methylfuran, 3-methylfuran, 2-ethylfuran, 2-propylfuran, 2-butylfuran, 2,3-dimethylfuran, 2,4-dimethylfuran, 2,5-dimethylfuran, pyran, 2-methylpyran, 3-methylpyran, 4-methylpyran, benzofuran, 2-(2-nitrovinyl)furan, or a mixture thereof, but is not limited thereto.

In an embodiment of the present disclosure, the thiophene-based compound may include, for example, thiophene, 2-methylthiophene, 2-ethylthiophene, 2-propylthiophene, 2-butylthiophene, 2,3-dimethylthiophene, 2,4-dimethylthiophene, 2,5-dimethylthiophene or a mixture thereof, but is not limited thereto.

In an embodiment of the present disclosure, in addition to the linear ether and the conjugated heterocyclic compound, the nonaqueous solvent may further include a non-conjugated cyclic ether. The non-conjugated cyclic ether may include, for example, at least one selected from the group consisting of 1,3-dioxolane, 4,5-dimethyl-dioxolane, 4,5-diethyl-dioxolane, 4-methyl-1,3-dioxolane, 4-ethyl-1,3-dioxolane, tetrahydrofuran, 2-methyltetrahydrofuran, 2,5-dimethyltetrahydrofuran, 2,5-dimethoxytetrahydrofuran, 2-ethoxytetrahydrofuran, 2-methyl-1,3-dioxolane, 2-vinyl-1,3-dioxolane, 2,2-dimethyl-1,3-dioxolane, 2-methoxy-1,3-dioxolane, 2-ethyl-2-methyl-1,3-dioxolane, tetrahydropyran, 1,4-dioxane, 1,2-dimethoxy benzene, 1,3-dimethoxy benzene, 1,4-dimethoxy benzene and isosorbide dimethyl ether. Preferably, the non-conjugated cyclic ether may include at least one selected from the group consisting of 1,3-dioxolane, tetrahydrofuran, 2-methyltetrahydrofuran and 2,5-dimethyltetrahydrofuran, is not limited thereto.

In an embodiment of the present disclosure, the nonaqueous solvent may include 1,2-dimethoxyethane and 2-methylfuran.

Additionally, the nonaqueous solvent may include the linear ether and the conjugated heterocyclic compound at a volume ratio of 95:5 to 5:95, preferably 95:5 to 50:50, and most preferably 90:10 to 70:30 or 80:20. The volume ratio is a ratio of "vol% of the linear ether":"vol% of the conjugated heterocyclic compound" in the nonaqueous solvent.

In an embodiment of the present disclosure, in addition to the linear ether, the conjugated heterocyclic compound and the non-conjugated cyclic ether compound, the nonaqueous solvent may further include any organic solvent that dissolves the lithium salt, the nitrate and the aryl derivative, for example, any organic solvent used in electrolyte solutions of lithium secondary batteries such as ester, amide, linear carbonate and cyclic carbonate. In an embodiment of the present disclosure, in addition to the ether-based solvent, the nonaqueous solvent may further include any nonaqueous solvent used in electrolyte solutions of lithium secondary batteries. However, preferably, in terms of solubility of the lithium salt, the nitrate and the aryl derivative, the electrolyte solution for the lithium secondary battery may not include the carbonate-based solvent as the nonaqueous solvent.

In an embodiment of the present disclosure, the ester may include, for example, any one selected from the group consisting of methyl acetate, ethyl acetate, propyl acetate, methyl propionate, ethyl propionate, propyl propionate, γ-butyrolactone, γ-valerolactone, γ-caprolactone, σ-valerolactone and ε-caprolactone or a mixture thereof, but is not limited thereto.

In an embodiment of the present disclosure, the linear carbonate may typically include, for example, any one selected from the group consisting of dimethyl carbonate, diethyl carbonate, dipropyl carbonate, ethylmethyl carbonate, methylpropyl carbonate and ethylpropyl carbonate or a mixture thereof, but is not limited thereto.

In an embodiment of the present disclosure, the cyclic carbonate includes any one selected from the group consisting of ethylene carbonate, propylene carbonate, 1,2-butylene carbonate, 2,3-butylene carbonate, 1,2-pentylene carbonate, 2,3-pentylene carbonate, vinylene carbonate, vinylethylene carbonate and halides thereof or a mixture thereof. Additionally, the halides may include, for example, fluoroethylene carbonate, but is not limited thereto.

In another embodiment of the present disclosure, because the carbonate-based solvent does not dissolve the nitrate and/or the aryl derivative or has a low solubility, the nonaqueous solvent may not include the carbonate-based solvent.

In an embodiment of the present disclosure, the nonaqueous solvent may include the carbonate-based solvent in such a small amount that the carbonate-based solvent does not affect the solubility of the nitrate and/or the aryl derivative, and for example, when the nonaqueous solvent includes the carbonate-based solvent, the amount of the carbonate-based solvent may be 3 wt% or less, 2 wt% or less, 1 wt% or less, 0.5 wt% or less or 0 wt% (i.e., zero) based on the total weight of the electrolyte solution for the lithium secondary battery.

In an aspect of the present disclosure, the lithium salt may include any lithium salt commonly used in electrolyte solutions of lithium-sulfur batteries without limitation. The lithium salt may include, for example, LiCl, LiBr, LiI, LiClO₄, LiBF₄, LiB₁₀Cl₁₀, LiPF₆, LiCF₃SO₃, LiCF₃CO₂, LiC₄BO₈, LiAsF₆, LiSbF₆, LiAlCl₄, CH₃SO₃Li, CF₃SO₃Li, (CF₃SO₂)₂NLi, (C₂F₅SO₂)₂NLi, (SO₂F)₂NLi, (CF₃SO₂)₃CLi, lithium chloroborate, lithium lower aliphatic carboxylate, lithium tetraphenylborate, lithium imide or a mixture thereof, but is not limited thereto.

In an embodiment of the present disclosure, the lithium salt may not include lithium nitrate. The lithium nitrate may be included as an example of the above-described nitrate.

In an embodiment of the present disclosure, the lithium salt may include lithium bis(fluorosulfonyl)imide (LiFSI).

In an embodiment of the present disclosure, the concentration of the lithium salt may be appropriately determined in view of ionic conductivity and solubility, and may be, for example, 0.1 to 4.0 M, 0.25 to 3.0 M, 0.5 to 2.0 M, 0.5 to 1.0 M or 0.5 to 0.75 M. When the concentration of the lithium salt is within the above-described range, it may have a beneficial effect on ionic conductivity and viscosity of the electrolyte solution, but is not limited thereto.

In another embodiment of the present disclosure, in addition to the above-described composition, the electrolyte solution for the lithium-sulfur battery may further include an additive to improve charge and discharge characteristics and flame retardancy. The present disclosure is not limited to a particular type of additive, but the additive may include, for example, pyridine, triethylphosphite, triethanolamine, ethylene diamine, n-glyme, hexamethyl phosphoric acid triamide, nitrobenzene derivatives, sulfur, quinone imine dyes, N-substituted oxazolidinone, N,N-substituted imidazolidine, ammonium salts, pyrrole, 2-methoxy ethanol, aluminum trichloride, fluoroethylene carbonate (FEC), propene sultone (PRS) and vinylene carbonate (VC).

The electrolyte solution for the lithium-sulfur battery according to an aspect of the present disclosure may be prepared by methods commonly used in the art, and the present disclosure is not limited to a particular method.

### Lithium-sulfur battery

A lithium-sulfur battery according to another aspect of the present disclosure includes the above-described electrolyte solution for the lithium-sulfur battery, and a positive electrode including a positive electrode active material and a negative electrode including a negative electrode active material. Specifically, the positive electrode active material includes elemental sulfur, a sulfur compound or a mixture thereof.

### Positive electrode

The positive electrode may include a positive electrode current collector and a positive electrode active material layer on one or two surfaces of the positive electrode current collector.

The positive electrode current collector is not limited to a particular type and may include any positive electrode current collector that supports the positive electrode active material and has high conductivity without causing any chemical change in the battery. For example, the positive electrode current collector may include copper, stainless steel, aluminum, nickel, titanium, palladium, sintered carbon, copper or stainless steel treated with carbon, nickel or silver on the surface, aluminum-cadmium alloys, etc.

The positive electrode current collector may have microtexture on the surface to increase the bonding strength with the positive electrode active material, and may come in different forms, for example, a film, a sheet, a foil, a mesh, a net, a porous body, a foam, a nonwoven, etc.

The positive electrode active material layer includes the positive electrode active material, and may further include a conductive material, a binder and an additive.

The positive electrode active material may include a sulfur-carbon composite including a porous carbon material and sulfur in at least part of the inside of the porous carbon material and the outer surface of the porous carbon material. Because sulfur included in the positive electrode active material does not have electrical conductivity itself, sulfur is used in combination with conductive materials such as carbon materials. Accordingly, the sulfur is included in the form of the sulfur-carbon composite.

In an embodiment of the present disclosure, the positive electrode active material may include elemental sulfur, a sulfur compound or a mixture thereof. Specifically, the positive electrode active material may include inorganic sulfur (S₈), lithium (poly)sulfide (Li₂Sₙ, 1≤n≤8), disulfide compounds, organic sulfur compounds, carbon-sulfur polymers ((C₂Sₓ)ₙ, x=2.5 to 50, 2≤n) or a mixture thereof. Preferably, the sulfur may be inorganic sulfur.

The sulfur-carbon composite includes the porous carbon material to provide skeletons for uniformly and stably immobilizing the sulfur and compensate for low electrical conductivity of the sulfur, thereby enhancing electrochemical reaction.

The porous carbon material may be generally produced by carbonization of various carbon precursors. The porous carbon material includes irregular pores therein. The average diameter of the pores may be in a range between 1 and 200 nm, and the porosity may be in a range between 10 and 90 vol% of the total volume of the porous carbon material. When the average diameter of the pores is less than the above-described range, the pore size is only at a molecular level, making sulfur loading impossible, and on the contrary, when the average diameter of the pores exceeds the above-described range, it is not suitable for the electrode manufacturing process due to low mechanical strength of the porous carbon material.

In an embodiment of the present disclosure, 'the average diameter of the pores' may be measured by known methods for measuring the pore diameter of a porous material, and the measurement method is not particularly limited. For example, the pore diameter may be measured by electron scanning microscopy (SEM), field emission electron microscopy or laser diffraction. The measurement using laser diffraction may be, for example, conducted using a commercially available laser diffraction particle size analyzer (for example, Microtrac MT 3000).

In an embodiment of the present disclosure, the 'porosity' refers to a ratio of the volume occupied by pores to the total volume of a structure in % unit, and may be used interchangeably with the terms pore ratio, void ratio, etc. In the present disclosure, the method for measuring the porosity is not particularly limited. According to an embodiment of the present disclosure, the porosity may be measured by the BET method using nitrogen gas, mercury (Hg) porosimetry or ASTM D2873.

The porous carbon material may be a spherical, rod-like, spiky, platy, tubular or bulky shape and is not limited to a particular shape, and may be in any shape commonly used in lithium-sulfur batteries.

The porous carbon material may include any porous carbon material having a porous structure and large specific surface area commonly used in the art. For example, the porous carbon material may include at least one selected from the group consisting of graphite; graphene; carbon black such as Denka black, acetylene black, Ketjen black, channel black, furnace black, lamp black, thermal black, etc.; carbon nanotubes (CNT) such as single-walled carbon nanotubes (SWCNT), multi-walled carbon nanotubes (MWCNT), etc.; carbon fibers such as graphite nanofibers (GNF), carbon nanofibers (CNF), activated carbon fibers (ACF), etc.; graphite such as natural graphite, artificial graphite, expandable graphite, etc.; and activated carbon, but is not limited thereto. Preferably, the porous carbon material may be carbon nanotubes.

In the sulfur-carbon composite according to the present disclosure, the sulfur may be disposed in at least one of the inside of the porous carbon material or the outer surface of the porous carbon material, and for example, may be present in an area of less than 100%, preferably 1 to 95%, and more preferably 40 to 96% of the inside of the porous carbon material and the outer surface of the porous carbon material. When the sulfur is present inside the porous carbon material and on the outer surface of the porous carbon material within the above-described range, maximum effect may be exhibited in terms of electron transport area and electrolyte wetting. Specifically, when the sulfur is thinly and uniformly loaded onto the above-described range of areas inside of the porous carbon material and on the outer surface of the porous carbon material, it may be possible to increase the electron transport contact area during charging and discharging. When the sulfur is located in the area corresponding to 100% of the inside of the porous carbon material and the outer surface of the porous carbon material, the porous carbon material is completely covered with the sulfur, resulting in poor electrolyte wetting and little or no contact, thereby failing to accept electrons and participate in electrochemical reaction.

The sulfur-carbon composite may, for example, include the sulfur in an amount of 65 wt% or more, specifically 65 to 90 wt%, 70 to 85 wt%, or 72 to 80 wt% based on 100 wt% of the sulfur-carbon composite. When the sulfur content is within the above-described range, it may have a beneficial effect on performance and capacity of the battery, but the present disclosure is not limited thereto.

The sulfur-carbon composite of the present disclosure may be produced by any method commonly used in the art without limitation. For example, the composite may be produced by simply mixing the sulfur with the porous carbon material and then heat-treating the mixture.

In an embodiment of the present disclosure, in addition to the above-described composition, the positive electrode active material may further include at least one additive selected from transition metal elements, Group IIIA elements, Group IVA elements, compounds of these elements with sulfur, and alloys of these elements with sulfur.

The transition metal elements may include Sc, Ti, V, Cr, Mn, Fe, Co, Ni, Cu, Zn, Y, Zr, Nb, Mo, Tc, Ru, Rh, Pd, Os, Ir, Pt, Au or Hg, the Group IIIA elements may include Al, Ga, In, Ti, etc., and the Group IVA elements may include Ge, Sn, Pb, etc.

The positive electrode for the lithium secondary battery of the present disclosure may include, for example, the positive electrode active material in an amount of 80 wt% or more, specifically 80 wt% to 100 wt%, and more specifically 85 wt% to 98 wt%, or 80 wt% to 95 wt% based on the total weight of the positive electrode active material layer. The lower limit of the amount of the positive electrode active material may be 70 wt% or more or 85 wt% or more based on the total 100 wt% of the positive electrode active material layer, and the upper limit may be 99 wt% or less or 90 wt% or less based on the total 100 wt% of the positive electrode active material layer. The amount of the positive electrode active material may be set by a combination of the lower limit and the upper limit. When the amount of the positive electrode active material is less than the above-described range, the relative quantity of the subsidiary materials such as the conductive material and the binder is high and the amount of the positive electrode active material is low, which makes it difficult to achieve the battery with high capacity and high energy density. On the contrary, when the amount of the sulfur-carbon composite exceeds the above-described range, the relative quantity of the conductive material or the binder as described below is low and the physical properties of the electrode decrease.

The conductive material acts as movement paths of electrons from the current collector to the positive electrode active material by electrically connecting the electrolyte to the positive electrode active material, and may include, without limitation, any material having conductive properties as the component of the electrode, which is physically different from the carbon contained in the sulfur-carbon composite.

For example, the conductive material may include carbon black such as Super-P, Denka black, acetylene black, Ketjen black, channel black, furnace black, lamp black, thermal black, carbon black, etc.; carbon derivatives such as carbon nanotubes, fullerene, etc.; conductive fibers such as carbon fibers, metal fibers, etc.; fluorocarbons; metal powders such as aluminum powder, nickel powder, etc.; or conductive polymers such as polyaniline, polythiophene, polyacetylene, polypyrrole, etc., used singly or in combination.

The amount of the conductive material may be 1 to 10 wt% based on the total weight of the positive electrode active material. When the amount of the conductive material is less than the above-described range, electron transfer between the positive electrode active material and the current collector reduces, resulting in lower voltage and capacity. On the contrary, when the amount of the conductive material exceeds the above-described range, the relative proportion of the positive electrode active material is low, and the total energy (amount of charges) of the battery may decrease. Therefore, it is preferable to determine an optimal amount within the above-described range.

The binder binds the positive electrode active material to the positive electrode current collector and interconnects the positive electrode active material to increase the bond strength, and may include any binder known in the art.

For example, the binder may include any one selected from the group consisting of a fluororesin-based binder including polyvinylidene fluoride (PVDF), a polyvinylidene fluoride-based polymer containing at least one vinylidene fluoride as a repeating unit, polytetrafluoroethylene (PTFE) or a mixture thereof; a rubber-based binder including styrene-butadiene rubber (SBR), acrylonitrile-butadiene rubber and styrene-isoprene rubber; an acrylic binder; a cellulose-based binder including carboxymethyl cellulose (CMC), starch, hydroxypropyl cellulose and regenerated cellulose; a polyalcohol-based binder; a polyolefin-based binder including polyethylene and polypropylene; a polyimide-based binder; a polyester-based binder; and a silane-based binder; and a mixture thereof or a copolymer thereof.

In an embodiment of the present disclosure, the binder may include polyacrylic acid (PAA).

The amount of the binder may be 1 to 10 wt% based on the total weight of the positive electrode active material layer. When the amount of the binder is less than the above-described range, the physical properties of the positive electrode may decrease and the positive electrode active material and the conductive material may be desorbed. When the amount of the binder exceeds the above-described range, the relative proportion of the positive electrode active material and the conductive material in the positive electrode is low, resulting in low battery capacity. Therefore, it is preferable to determine an optimal amount within the above-described range.

In the present disclosure, a method for manufacturing the positive electrode for the lithium secondary battery is not particularly limited, and may include any method known to those skilled in the art or its variations.

By way of example, the positive electrode for the lithium secondary battery may be manufactured by preparing a positive electrode slurry composition comprising the above-described composition and coating the positive electrode slurry composition on at least one surface of the positive electrode current collector to form the positive electrode active material layer.

The positive electrode slurry composition comprises the positive electrode active material described above and may further comprise the binder, the conductive material and a solvent.

The solvent includes any solvent that facilitates the uniform dispersion of the positive electrode active material. The solvent may include a water-based solvent, most preferably water, and in this instance, water may be distilled water or deionized water. However, the present disclosure is not necessarily limited thereto and if necessary, lower alcohol that easily mixes with water may be used. The lower alcohol may include methanol, ethanol, propanol, isopropanol and butanol, and preferably, a mixture of lower alcohol and water may be used.

The solvent may be included at a sufficient level of concentration to make coating easy, and the specific amount may change depending on the coating method and device.

The positive electrode slurry composition may further comprise a material commonly used to improve functions in the corresponding technical field, if necessary. For example, the material may include a viscosity modifier, a glidant, a filler, etc.

A method for coating the positive electrode slurry composition is not particularly limited in the present disclosure, and for example, may include doctor blade, die casting, comma coating, screen printing, etc. In addition, after forming on a separate substrate, the positive electrode slurry may be coated on the positive electrode current collector by pressing or lamination.

After the coating, a drying process may be performed to remove the solvent. The drying process may be performed at suitable temperatures and times to remove the solvent, and the conditions may change depending on the type of the solvent and are not particularly limited in the present disclosure. By way of example, the drying method may include drying with warm air, hot air or low-humidity air, vacuum drying, drying by (far)infrared radiation or electron beam radiation, etc. The drying speed is usually adjusted to remove the solvent as quickly as possible within a speed range in which cracking does not occur in the positive electrode active material layer or the positive electrode active material layer is not peeled off from the positive electrode current collector due to stress concentration.

In addition, after the drying, the current collector may be pressed to increase the density of the positive electrode active material in the positive electrode. The pressing method may include mold pressing, roll pressing, etc.

The positive electrode manufactured by the above-described composition and manufacturing method, specifically the positive electrode active material layer, may have the porosity of 50 to 80 vol%, specifically 60 to 75 vol%. When the porosity of the positive electrode is less than 50 vol%, the filling level of the positive electrode slurry composition comprising the positive electrode active material, the conductive material and the binder is too high, thereby failing to maintain enough electrolyte to exhibit ionic conductivity and/or electrical conductivity in the positive electrode active material. As a result, the output characteristics or cycle characteristics of the battery may degrade, which induces severe overvoltage and reduction in discharge capacity of the battery. On the contrary, when the porosity of the positive electrode is too high over 80 vol%, it may result in poor physical and electrical connection with the current collector, low adhesion strength and no or little reaction, and the filling of the electrolyte at the high porosity may reduce the energy density of the battery. Accordingly, the porosity is appropriately adjusted within the above-described range.

### Negative electrode

The negative electrode may include a negative electrode current collector and a negative electrode active material layer on one or two surfaces of the negative electrode current collector. Alternatively, the negative electrode may be a lithium metal plate.

The negative electrode current collector is configured to support the negative electrode active material layer, as described in the positive electrode current collector.

The negative electrode active material layer may include a negative electrode active material, and may further include a conductive material, a binder, etc. In this instance, the conductive material and the binder follow the above description.

The negative electrode active material may include materials capable of reversibly intercalating or deintercalating lithium (Li⁺), materials capable of reacting with lithium ions to reversibly form lithium containing compounds, lithium metals or lithium alloys.

The materials capable of reversibly intercalating or deintercalating lithium ions (Li⁺) may include, for example, crystalline carbon, amorphous carbon or a mixture thereof. The materials capable of reacting with lithium ions (Li⁺) to reversibly form lithium containing compounds may include, for example, tin oxide, titanium nitrate or silicon. The lithium alloys may include, for example, alloys of lithium (Li) and metal selected from the group consisting of sodium (Na), potassium (K), rubidium (Rb), cesium (Cs), francium (Fr), beryllium (Be), magnesium (Mg), calcium (Ca), strontium (Sr), barium (Ba), radium (Ra), aluminum (Al) and tin (Sn).

Preferably, the negative electrode active material may be a lithium metal, and specifically, may be in the form of a lithium metal foil or a lithium metal powder.

### Separator

The separator separates or insulates the positive electrode from the negative electrode and enables the transport of lithium ions between the positive electrode and the negative electrode. The separator may be made of a porous nonconductive or insulating material and is not limited to a particular type and may include any separator commonly used in lithium secondary batteries. The separator may be a stand-alone member such as a film, and may also be a coating layer added to the positive electrode and/or the negative electrode.

The separator may preferably have low resistance to the movement of electrolyte ions and high ability to absorb the electrolyte.

The separator may include a porous substrate, and the porous substrate may include any porous substrate commonly used in secondary batteries, and a porous polymer film may be used alone or in a stack. For example, a nonwoven fabric made of high-melting-point glass fibers, polyethylene terephthalate fibers, etc. or a polyolefin-based porous membrane may be used, but is not limited thereto.

The porous substrate is not limited to a particular material in the present disclosure, and may include any porous substrate commonly used in electrochemical devices. For example, the porous substrate may include at least one type of material selected from the group consisting of polyolefin such as polyethylene, polypropylene, etc., polyester such as polyethylene terephthalate, polybutylene terephthalate, etc., polyamide, polyacetal, polycarbonate, polyimide, polyether ether ketone, polyethersulfone, polyphenylene oxide, polyphenylene sulfide, polyethylene naphthalate, polytetrafluoroethylene, polyvinylidene fluoride, polyvinyl chloride, polyacrylonitrile, cellulose, nylon, poly(p-phenylene benzobisoxazole) and polyarylate.

The thickness of the porous substrate may be 1 to 100 µm, and preferably 5 to 50 µm, but is not limited thereto. Although the thickness of the porous substrate is not limited to the above-described range, when the thickness is too small below the above-described lower limit, the mechanical properties may decrease and the separator may be easily damaged while the battery is in use.

The porous substrate may have an average pore diameter of 0.001 to 50 µm and a porosity 10 to 95 vol%, but is not limited thereto.

The lithium-sulfur battery according to the present disclosure may allow the commonly used winding process as well as lamination or stacking and folding processes of the separator and the electrode.

The lithium-sulfur battery is not limited to a particular shape and may come in different shapes, for example, cylindrical, stack-type, coin-type, pouch-type, etc.

Hereinafter, exemplary embodiments are presented to help understanding of the present disclosure, but the following embodiments describe the present disclosure by way of illustration only and it is obvious to those skilled in the art that a variety of modifications and changes may be made within the scope and technical aspect of the present disclosure, and such modifications and changes fall within the scope of the appended claims.

### [Manufacture of lithium-sulfur battery]

### Example 1

### Preparation of electrolyte solution for lithium-sulfur battery

0.5 M of lithium bis(fluorosulfonyl)imide (LiFSI), 4 wt% of lithium nitrate (LiNO₃, molar mass 69 g/mol) and 1 wt% of dipyridyl disulfide (PyDS, molar mass 220.31 g/mol) were added to a mixture of 1,2-dimethoxyethane (DME) and 2-methylfuran (2MeF) (8:2 v/v) and stirred at room temperature (23°C) for 12 hours to prepare an electrolyte solution.

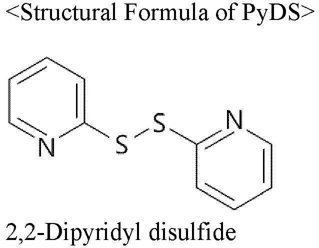

### Preparation of electrode

A sulfur-carbon composite (S₈:CNT=70:30 (weight ratio)) as a positive electrode active material and polyacrylic acid (PAA) as a binder were mixed at a weight ratio of 96:4 in distilled water as a solvent to prepare a positive electrode slurry.

The positive electrode slurry was coated on two surfaces of a 12 µm thick aluminum current collector, dried at 80°C and rolled using a roll press machine to make a positive electrode. In this instance, the loading amount of the positive electrode active material was 3.3 mAh/cm², and the porosity of the positive electrode active material layer excluding the current collector from the positive electrode was 74 vol%.

For a negative electrode, a 50 µm thick lithium metal foil was prepared.

### Manufacture of lithium-sulfur battery

The positive electrode and the negative electrode as prepared above were placed with a polyethylene separator having a thickness of 16 µm and a porosity of 68 vol% interposed between and stacked together to assemble a pouch cell, and 1g of the prepared electrolyte solution was injected such that a ratio of Electrolyte/S loading amount (El/S) is 2.5 g/g, followed by sealing to fabricate a pouch-type lithium-sulfur battery.

### Example 2

A lithium-sulfur battery was manufactured by the same method as Example 1 except that 3 wt% of dipyridyl disulfide (PyDS) was added.

### Comparative Example 1

A lithium-sulfur battery was manufactured by the same method as Example 1 except that dipyridyl disulfide (PyDS) was not added.

### Comparative Example 2

An electrolyte solution was prepared by the same method as Example 1 except that for the nonaqueous solvent, DME:2-MeF (8:2 v/v) was replaced with ethylenecarbonate (EC):dimethylcarbonate (DMC)(1:2 v/v). In Comparative Example 2, it was confirmed that lithium nitrate (LiNO₃) did not dissolve and could not be used as the electrolyte solution for the lithium-sulfur battery.

### Comparative Example 3

A lithium-sulfur battery was manufactured by the same method as Example 1 except that for the nonaqueous solvent, DME:2-MeF (8:2 v/v) was replaced with 1,2-dimethoxyethane (DME):1,3-dioxolane(1,3-dioxolane, DOL)(5:5 v/v).

### Comparative Example 4

A lithium-sulfur battery was manufactured by the same method as Example 1 except that for the nonaqueous solvent, DME:2-MeF (8:2 v/v) was replaced with 1,2-dimethoxyethane (DME):2-methyltetrahydrofuran (2-Me-THF)(8:2 v/v).

### Experimental Example 1. Battery performance evaluation and results

For each of the batteries of Example 1, Example 2 and Comparative Example 1, performance evaluation was performed by the following method. In the case of Comparative Example 2, because nitrate in the electrolyte solution did not dissolve as described above, its battery performance evaluation result was omitted.

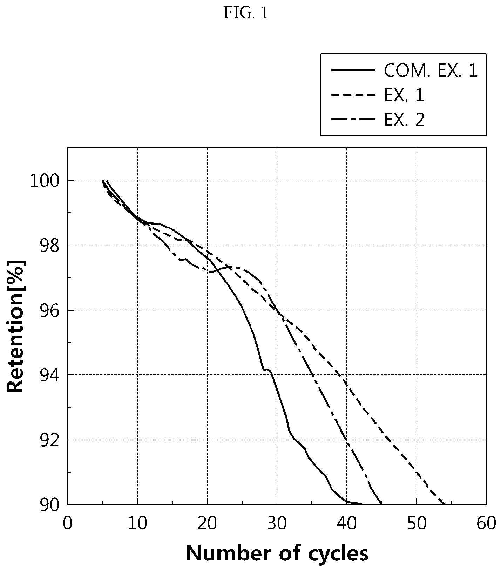

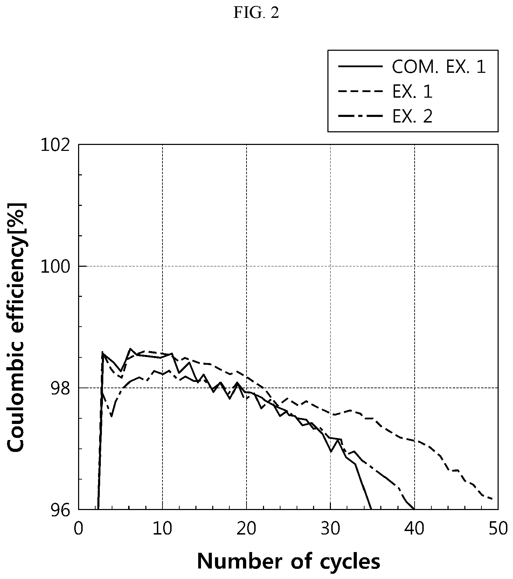

Battery performance was evaluated by repeating 3 charge and discharge cycles of 0.1C charge/0.1C discharge in 1.8 V to 2.5 V cut-off condition at 25°C, followed by 0.2C charge/0.1C discharge, and FIGs. 1 and 2 show the results.

FIG. 1 shows the number of cycles until the capacity that fades over repeated charge and discharge cycles reaches 90% given that capacity in the fifth cycle after capacity stabilization is 100%.

Referring to FIG. 1, it was confirmed that 90% capacity retention was reached in 54 cycles for Example 1, 45 cycles for Example 2, and 40 cycles for Comparative Example 1.

Through the experiment, it was confirmed that the batteries of Example 1 and Example 2 suppressed capacity fading and improved capacity retention over repeated charge and discharge cycles and 90% capacity retention was reached at a later time than Comparative Example 1.

FIG. 2 shows changes in Coulombic efficiency per weight over repeated charge and discharge cycles.

Referring to FIG. 2, it was confirmed that the lithium-sulfur battery reduced in Coulombic efficiency rapidly over repeated charge and discharge cycles due to the shuttle effect of lithium polysulfide. In this instance, the batteries of Example 1 and Example 2 using PyDS in the electrolyte solution showed a notable delay in Coulombic efficiency reduction compared to the battery of Comparative Example 1 without PyDS.

### Experimental Example 2. Battery performance evaluation and results

For each of the batteries of Example 1, Comparative Example 3 and Comparative Example 4, performance evaluation was performed by the following method.

Battery performance was evaluated by repeating 3 charge and discharge cycles of 0.1C charge/0.1C discharge in 1.8 V to 2.5 V cut-off condition at 25°C, followed by 0.2C charge/0.1C discharge, and FIGs. 1 and 2 show the results.

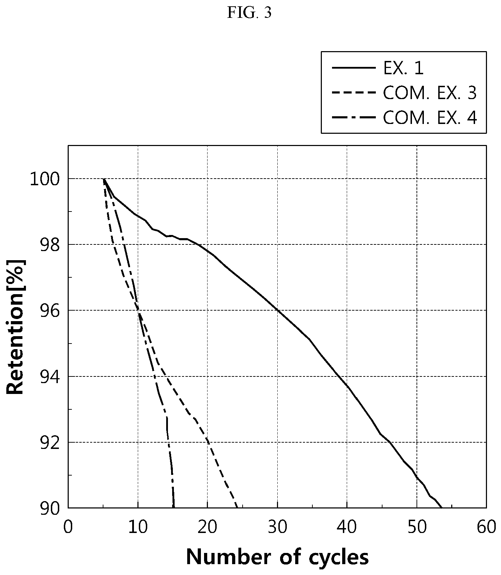

FIG. 3 shows the number of cycles until the capacity that fades over repeated charge and discharge cycles reaches 90% given that capacity in the fifth cycle after capacity stabilization is 100%.

Referring to FIG. 3, it was confirmed that 90% capacity retention was reached in 54 cycles for Example 1, 24 cycles for Comparative Example 3, and 15 cycles for Comparative Example 4.

In this instance, Comparative Example 3 and Comparative Example 4 include the aryl derivative according to an embodiment of the present disclosure as the electrolyte solution composition, but include the non-conjugated heterocyclic compound instead of the conjugated heterocyclic compound as the nonaqueous solvent.

Through the experiment, it was confirmed that when the aryl derivative according to an embodiment of the present disclosure is included in the electrolyte solution of the lithium-sulfur battery, the conjugated heterocyclic compound such as 2-methyl furan should be included as the nonaqueous solvent.

## Claims

1. An electrolyte solution for a lithium-sulfur battery, comprising:
a nonaqueous solvent, a lithium salt, nitrate and an aryl derivative,
wherein the nonaqueous solvent includes a linear ether compound and a conjugated heterocyclic compound, and
wherein the aryl derivative includes a compound represented by the following Chemical Formula 1, a compound represented by the following Chemical Formula 2, a compound represented by the following Chemical Formula 3 or a mixture thereof:
[Chemical Formula 1] R₁-S⁻
[Chemical Formula 2] R₂-S-H
[Chemical Formula 3] R₃-S-S-R₄
wherein in the Chemical Formulas 1 to 3, each of R₁ to R₄ is independently a 5- or 6-membered heteroaryl containing at least one N or S atom as a ring atom.

2. The electrolyte solution for the lithium-sulfur battery according to claim 1, wherein each of R₁ to R₄ is independently an ortho-pyridyl group.

3. The electrolyte solution for the lithium-sulfur battery according to claim 1, wherein the aryl derivative includes 2,2'-dipyridyl disulfide.

4. The electrolyte solution for the lithium-sulfur battery according to claim 1, wherein the aryl derivative is included in an amount of 0.1 wt% to 5 wt% based on a total weight of the electrolyte solution for the lithium-sulfur battery.

5. The electrolyte solution for the lithium-sulfur battery according to claim 1, wherein the aryl derivative is included in an amount of 1 wt% to 3 wt% based on a total weight of the electrolyte solution for the lithium-sulfur battery.

6. The electrolyte solution for the lithium-sulfur battery according to claim 1, wherein a weight ratio of the aryl derivative to the nitrate (the aryl derivative/the nitrate) is 0.2 to 1.5.

7. The electrolyte solution for the lithium-sulfur battery according to claim 1, wherein the linear ether compound and the conjugated heterocyclic compound are included in an amount of 80 vol% or more based on a total weight of the nonaqueous solvent.

8. The electrolyte solution for the lithium-sulfur battery according to claim 1, wherein the conjugated heterocyclic compound is a conjugated cyclic ether compound.

9. The electrolyte solution for the lithium-sulfur battery according to claim 1, wherein the nonaqueous solvent does not include a carbonate-based solvent.

10. The electrolyte solution for the lithium-sulfur battery according to claim 1, wherein a cation of the nitrate is an alkali metal.

11. A lithium-sulfur battery comprising:
a positive electrode; a negative electrode; a separator between the positive electrode and the negative electrode; and the electrolyte solution according to any one of claims 1 to 10.

12. The lithium-sulfur battery according to claim 11, wherein the positive electrode includes a sulfur-containing compound as a positive electrode active material.

13. The lithium-sulfur battery according to claim 12, wherein the sulfur-containing compound includes an inorganic sulfur (S₈), lithium (poly)sulfide (Li₂Sn, 1≤n≤8), a carbon-sulfur polymer ((C₂Sx)m, 2.5≤x≤50, 2≤m) or a mixture thereof.

14. The lithium-sulfur battery according to claim 11, wherein the negative electrode includes a lithium metal, a lithium alloy or a mixture thereof as a negative electrode active material.

15. The lithium-sulfur battery according to claim 11, wherein the lithium-sulfur battery is a coin-type battery, a pouch-type battery or a cylindrical battery.

## Patentansprüche

1. Elektrolytlösung für eine Lithium-Schwefel-Batterie, umfassend:
ein nichtwässriges Lösungsmittel, ein Lithiumsalz, Nitrat und ein Arylderivat,
wobei das nichtwässrige Lösungsmittel eine lineare Etherverbindung und eine konjugierte heterocyclische Verbindung umfasst, und
wobei das Arylderivat eine durch die folgende chemische Formel 1 dargestellte Verbindung, eine durch die folgende chemische Formel 2 dargestellte Verbindung, eine durch die folgende chemische Formel 3 dargestellte Verbindung oder eine Mischung davon umfasst:
[Chemische Formel 1] R₁-S⁻
[Chemische Formel 2] R₂-S-H
[Chemische Formel 3] R₃-S-S-R₄
wobei in den Chemischen Formeln 1 bis 3 jedes von R₁ bis R₄ unabhängig ein 5- oder 6-gliedriges Heteroaryl ist, das mindestens ein N- oder S-Atom als ein Ringatom enthält.

2. Elektrolytlösung für die Lithium-Schwefel-Batterie gemäß Anspruch 1, wobei jedes von R₁ bis R₄ unabhängig eine ortho-Pyridylgruppe ist.

3. Elektrolytlösung für die Lithium-Schwefel-Batterie gemäß Anspruch 1, wobei das Arylderivat 2,2'-Dipyridyldisulfid umfasst.

4. Elektrolytlösung für die Lithium-Schwefel-Batterie gemäß Anspruch 1, wobei das Arylderivat in einer Menge von 0,1 Gew.-% bis 5 Gew.-%, bezogen auf ein Gesamtgewicht der Elektrolytlösung für die Lithium-Schwefel-Batterie, enthalten ist.

5. Elektrolytlösung für die Lithium-Schwefel-Batterie gemäß Anspruch 1, wobei das Arylderivat in einer Menge von 1 Gew.-% bis 3 Gew.-%, bezogen auf ein Gesamtgewicht der Elektrolytlösung für die Lithium-Schwefel-Batterie, enthalten ist.

6. Elektrolytlösung für die Lithium-Schwefel-Batterie gemäß Anspruch 1, wobei ein Gewichtsverhältnis des Arylderivats zu dem Nitrat (das Arylderivat/das Nitrat) 0,2 bis 1,5 beträgt.

7. Elektrolytlösung für die Lithium-Schwefel-Batterie gemäß Anspruch 1, wobei die lineare Etherverbindung und die konjugierte heterocyclische Verbindung in einer Menge von 80 Vol.-% oder mehr, bezogen auf ein Gesamtgewicht des nichtwässrigen Lösungsmittels, enthalten sind.

8. Elektrolytlösung für die Lithium-Schwefel-Batterie gemäß Anspruch 1, wobei die konjugierte heterocyclische Verbindung eine konjugierte cyclische Etherverbindung ist.

9. Elektrolytlösung für die Lithium-Schwefel-Batterie gemäß Anspruch 1, wobei das nichtwässrige Lösungsmittel kein Lösungsmittel auf Carbonatbasis umfasst.

10. Elektrolytlösung für die Lithium-Schwefel-Batterie gemäß Anspruch 1, wobei ein Kation des Nitrats ein Alkalimetall ist.

11. Lithium-Schwefel-Batterie, umfassend:
eine positive Elektrode; eine negative Elektrode; einen Separator zwischen der positiven Elektrode und der negativen Elektrode; und die Elektrolytlösung gemäß einem der Ansprüche 1 bis 10.

12. Lithium-Schwefel-Batterie gemäß Anspruch 11, wobei die positive Elektrode eine schwefelhaltige Verbindung als aktives Material der positiven Elektrode umfasst.

13. Lithium-Schwefel-Batterie gemäß Anspruch 12, wobei die schwefelhaltige Verbindung einen anorganischen Schwefel (S₈), Lithium(poly)sulfid (Li₂Sn, 1≤n≤8), ein Kohlenstoff-Schwefel-Polymer ((C₂Sx)m, 2,5≤x≤50, 2≤m) oder eine Mischung davon umfasst.

14. Lithium-Schwefel-Batterie gemäß Anspruch 11, wobei die negative Elektrode ein Lithiummetall, eine Lithiumlegierung oder eine Mischung davon als aktives Material der negativen Elektrode umfasst.

15. Lithium-Schwefel-Batterie gemäß Anspruch 11, wobei die Lithium-Schwefel-Batterie eine Batterie vom Münztyp, eine Batterie vom Beuteltyp oder eine zylindrische Batterie ist.

## Revendications

1. Solution d'électrolytes pour une batterie lithium-soufre, comprenant :
un solvant non aqueux, un sel de lithium, du nitrate et un dérivé d'aryle,
dans laquelle le solvant non aqueux comprend un composé d'éther linéaire et un composé hétérocyclique conjugué, et
dans laquelle le dérivé d'aryle comprend un composé représenté par la Formule Chimique 1 suivante, un composé représenté par la Formule Chimique 2 suivante, un composé représenté par la Formule Chimique 3 suivante ou un mélange de ceux-ci :
[Formule Chimique 1] R₁-S⁻
[Formule Chimique 2] R₂-S-H
[Formule Chimique 3] R₃-S-S-R₄
dans laquelle dans les Formules Chimiques 1 à 3, chacun de R₁ à R₄ est indépendamment un hétéroaryle à 5 ou 6 chaînons contenant au moins un atome N ou S en tant qu'atome cyclique.

2. Solution d'électrolytes pour la batterie lithium-soufre selon la revendication 1, dans laquelle chacun de R₁ à R₄ est indépendamment un groupe ortho-pyridyle.

3. Solution d'électrolytes pour la batterie lithium-soufre selon la revendication 1, dans laquelle le dérivé d'aryle comprend du disulfure de 2,2'-dipyridyle.

4. Solution d'électrolytes pour la batterie lithium-soufre selon la revendication 1, dans laquelle le dérivé d'aryle est inclus en une quantité de 0,1 % en poids à 5 % en poids sur la base du poids total de la solution d'électrolytes pour la batterie lithium-soufre.

5. Solution d'électrolytes pour la batterie lithium-soufre selon la revendication 1, dans laquelle le dérivé d'aryle est inclus en une quantité de 0,1 % en poids à 3 % en poids sur la base du poids total de la solution d'électrolytes pour la batterie lithium-soufre.

6. Solution d'électrolytes pour la batterie lithium-soufre selon la revendication 1, dans laquelle un rapport pondéral du dérivé d'aryle au nitrate (le dérivé d'aryle/nitrate) est de 0,2 à 1,5.

7. Solution d'électrolytes pour la batterie lithium-soufre selon la revendication 1, dans laquelle le composé d'éther linéaire et le composé hétérocyclique conjugué sont inclus en une quantité de 80 % en volume sur la base du poids total du solvant non aqueux.

8. Solution d'électrolytes pour la batterie lithium-soufre selon la revendication 1, dans laquelle le composé hétérocyclique conjugué est un composé d'éther cyclique conjugué.

9. Solution d'électrolytes pour la batterie lithium-soufre selon la revendication 1, dans laquelle le solvant non aqueux ne comprend pas de solvant à base de carbonate.

10. Solution d'électrolytes pour la batterie lithium-soufre selon la revendication 1, dans laquelle un cation du nitrate est un métal alcalin.

11. Batterie lithium-soufre comprenant :
une électrode positive ; une électrode négative ; un séparateur entre l'électrode positive et l'électrode négative ; et la solution d'électrolytes selon l'une quelconque des revendications 1 à 10.

12. Batterie lithium-soufre selon la revendication 11, dans laquelle l'électrode positive comprend un composé contenant du soufre en tant que matériau actif d'électrode positive.

13. Batterie lithium-soufre selon la revendication 12, dans laquelle le composé contenant du soufre comprend un soufre inorganique (S₈), du polysulfure de lithium (Li₂Sn, 1≤n≤8), un polymère de carbone-soufre ((C₂Sx)m, 2,5≤x≤50, 2≤m) ou un mélange de ceux-ci.

14. Batterie lithium-soufre selon la revendication 11, dans laquelle l'électrode négative comprend un métal de lithium, un alliage de lithium ou un mélange de ceux-ci en tant que matériau actif d'électrode négative.

15. Batterie lithium-soufre selon la revendication 11, où la batterie lithium-soufre est une pile bouton, une cellule poche ou une pile cylindrique.
